# EUROPEAN PATENT SPECIFICATION

(11) **EP 4 081 100 B1**
(45) Date of publication and mention of the grant of the patent: **29.10.2025**
(21) Application number: 20829227.6
(22) Date of filing: 23.12.2020
(51) Int. Cl.: A61B 5/00, A61M 16/00, A61M 16/06

(54) **SYSTEMS AND METHODS FOR GENERATING REMINDERS TO USE RESPIRATORY THERAPY SYSTEMS**
SYSTEME UND VERFAHREN ZUR ERZEUGUNG VON ERINNERUNGEN ZUR VERWENDUNG VON ATEMTHERAPIESYSTEMEN
SYSTÈMES ET PROCÉDÉS POUR GÉNÉRER DES RAPPELS POUR UTILISER DES SYSTÈMES DE THÉRAPIE RESPIRATOIRE

(30) Priority: 26.12.2019 US 201962953798 P
(43) Date of publication of application: 02.11.2022
(73) Proprietor: ResMed Sensor Technologies Limited, Dublin D18 T6T7 (IE)
(72) Inventor: COFFEY, Sam, Dublin, D18 T6T7 (IE); GRENNAN, Kieran, Dublin, D18 T6T7 (IE); O'MAHONY, Niall, Dublin, D18 T6T7 (IE); SHOULDICE, Redmond, Dublin, D18 T6T7 (IE)
(74) Representative: Mathys & Squire
(86) International application number: PCT/IB2020/062365
(87) International publication number: WO 2021/130681

(56) References cited:
- WO-A1-2015/020536
- WO-A1-2015/196255
- WO-A1-2017/027906
- WO-A2-2015/006364
- US-A1- 2019 076 100

## Description

### TECHNICAL FIELD

The present disclosure relates to treatment of respiratory-related disorders and more specifically to systems and methods for determining sleeping patterns of a user and generating alarms based on the user failing to adequately use a respiratory therapy system.

### BACKGROUND

Systems disclosed in US 2019/076100 A1, WO 2017/027906 A1, WO 2015/196255 A1, WO 2015/020536 A1, and WO 2015/006364 A2 aim at improving the sleep of a user. Various systems exist for aiding users experiencing sleep apnea and related respiratory disorders. A range of respiratory disorders exist that can impact users. Certain disorders are characterized by particular events (e.g., apneas, hypopneas, hyperpneas, or any combination thereof). Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and Chest wall disorders. A person with respiratory disorder can have trouble sleeping and may awake one or more times during a sleep session. Systems designed to mitigate physical symptoms of the respiratory disorder can require a level of engagement from the person prior to the person falling asleep. Sometimes depending on the person's state of mind, the person can forget to engage with the system prior to falling asleep. Thus, a need exists for alternative systems and methods for providing reminders to the person such that a desired level of engagement is elicited. The present disclosure is directed to solving these and other problems.

### SUMMARY

According to some implementations of the present disclosure, a method includes generating, by a sensor, data including current data that is associated with a current sleep session of a plurality of sleep sessions and historical data that is associated with one or more prior sleep sessions of the plurality of sleep sessions. The historical data is analyzed to determine a behavior pattern associated with a user. The current data is analyzed to determine a condition of a user interface in relation to the user, wherein the user interface is coupled to a respiratory therapy device configured to supply pressurized air to an airway of the user during the plurality of sleep sessions. An alarm is generated based at least in part on the behavior pattern associated with the user and the condition of the user interface in relation to the user.

According to some implementations of the present disclosure, a method includes generating, by a sensor, data including current data that is associated with a current sleep session of a plurality of sleep sessions and historical data that is associated with one or more prior sleep sessions of the plurality of sleep sessions. The historical data is analyzed to determine a sleeping pattern of a user, the sleeping pattern of the user including user behavior and circumstances that occurred prior to the current data being generated by the sensor, wherein a user interface is coupled to a respiratory therapy device configured to supply pressurized air to an airway of the user during at least a portion of the plurality of sleep sessions. A likelihood that the user will fall asleep within a first predetermined amount of time is determined, based at least in part on the current data and the determined sleeping pattern. An alarm is generated based at least in part on the determined likelihood and on the determined sleeping pattern of the user.

According to some implementations of the present disclosure, a method includes generating, by a sensor, data. The generated data is used to determine that a user is currently asleep. The generated data is used to determine that a user interface is not currently engaging the user, the user interface being coupled to a respiratory therapy device configured to supply pressurized air to an airway of the user. An alarm is generated to aid in alerting the user to don the user interface.

According to some implementations of the present disclosure, a system includes a respiratory therapy device, a sensor, one or more memory devices, and a control system. The respiratory therapy device is configured to supply, by way of a user interface coupled to the respiratory therapy device via a conduit, pressurized air to an airway of a user during a plurality of sleep sessions. The sensor is configured to generate data. The one or more memory devices store machine-readable instructions and at least a portion of the data generated by the sensor. The stored data includes current data that is associated with a current sleep session of the plurality of sleep sessions and historical data that is associated with one or more prior sleep sessions of the plurality of sleep sessions. The control system is configured to execute the machine-readable instructions to: (a) analyze the historical data to determine a behavior pattern associated with the user; (b) analyze the current data to determine a condition of the user interface in relation to the user; and (c) generate an alarm based at least in part on the behavior pattern associated with the user and the condition of the user interface in relation to the user.

According to some implementations of the present disclosure, a system includes a respiratory therapy device, a sensor, one or more memory devices, and a control system. The respiratory therapy device is configured to supply, by way of a user interface coupled to the respiratory therapy device via a conduit, pressurized air to an airway of a user during a plurality of sleep sessions. The sensor is configured to generate data. The one or more memory devices store machine-readable instructions and at least a portion of the data generated by the sensor. The stored data includes current data that is associated with a current sleep session of the plurality of sleep sessions and historical data that is associated with one or more prior sleep sessions of the plurality of sleep sessions. The control system includes one or more processors configured to execute the machine-readable instructions to: (a) analyze the historical data to determine a sleeping pattern of the user, the sleeping pattern of the user including user behavior and circumstances that occurred prior to current data being generated by the sensor; (b) determine, based at least in part on the current data and the determined sleeping pattern, a likelihood that the user will fall asleep within a first predetermined amount of time; and (c) generate an alarm based at least in part on the determined likelihood and on the determined sleeping pattern of the user.

According to some implementations of the present disclosure, a system includes a respiratory therapy device, a sensor, one or more memory devices, and a control system. The respiratory therapy device is configured to supply, by way of a user interface coupled to the respiratory therapy device via a conduit, pressurized air to an airway of a user during a plurality of sleep sessions. The sensor is configured to generate data. The one or more memory devices store machine-readable instructions. The control system includes one or more processors configured to execute the machine-readable instructions to: (a) determine, based at least in part on the generated data, that the user is currently asleep; (b) determine, based at least in part on the generated data, that the user interface is not currently engaging the user; and (c) generate an alarm to aid in alerting the user to don the user interface.

The above summary is not intended to represent each embodiment or every aspect of the present invention. Additional features and benefits of the present invention are apparent from the detailed description and figures set forth below.

### BRIEF DESCRIPTION OF THE DRAWINGS

FIG. 1 illustrates an environment for using a respiratory therapy system, according to some implementations of the present disclosure;
FIG. 2 is a block diagram of a system for generating an alarm based on data associated with a user of a respiratory therapy system, according to some implementations of the present disclosure;
FIG. 3 is a flow diagram for generating an alarm based at least in part on data associated with the user of the respiratory therapy system, according to some implementations of the present disclosure;
FIG. 4 is a flow diagram for generating an alarm based at least in part on a likelihood that the user of the respiratory therapy system will fall asleep, according to some implementations of the present disclosure;
FIG. 5 is a flow diagram for generating an alarm based at least in part on data associated with the user of the respiratory therapy system, according to some implementations of the present disclosure;
FIG. 6 illustrates an exemplary timeline for a sleep session, according to some implementations of the present disclosure; and
FIG. 7 illustrates an exemplary hypnogram associated with the sleep session of FIG. 3, according to some implementations of the present disclosure.

While the present disclosure is susceptible to various modifications and alternative forms, specific implementations and embodiments thereof have been shown by way of example in the drawings and will herein be described in detail.

### DETAILED DESCRIPTION

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB), Obstructive Sleep Apnea (OSA), apneas, Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as central apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathin. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

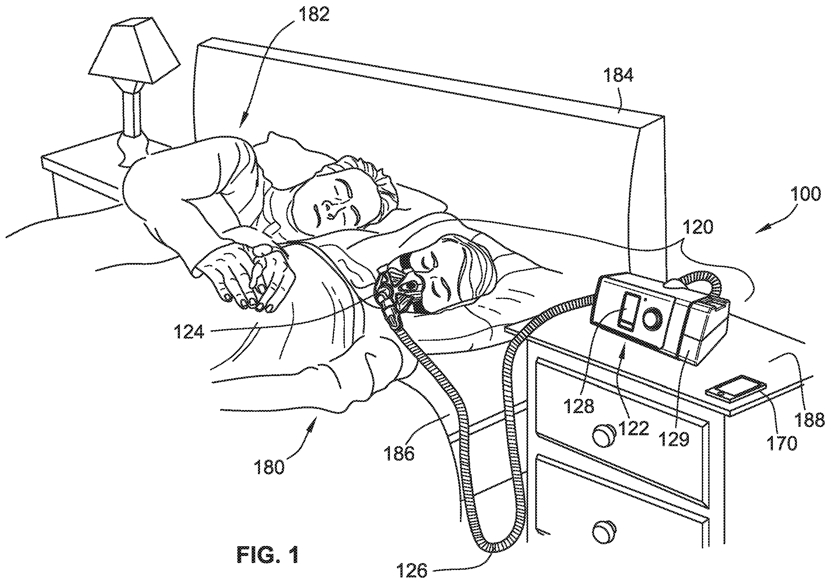

Respiratory therapy devices (or respiratory devices) can be used to aid users experiencing respiratory disorders. Respiratory therapy devices are unhelpful and unable to provide therapeutic relief if users do not actually use the respiratory therapy devices while sleeping. Referring to FIG. 1, a system 100, according to some implementations of the present disclosure, is illustrated. The system 100 is for generating alarms for reminding a user 180 to take advantage of a respiratory therapy system 120 (e.g., a respiratory pressure therapy system).

In some implementations, the respiratory therapy system 120 is collectively formed by a respiratory therapy device 122, a user interface 124 (also referred as a patient interface or a mask), a conduit 126 (also referred to as a tube or an air circuit), a display device 128, and a humidification tank 129. As shown, the user 180 of the respiratory therapy system 120 and a bed partner 182 are located in a bed 184 and are laying on a mattress 186. The user interface 124 (e.g., a full facial mask) can be worn by the user 180 during a sleep session. The user interface 124 is fluidly coupled and/or connected to the respiratory therapy device 122 via the conduit 126. In turn, the respiratory therapy device 122 delivers pressurized air to the user 180 via the conduit 126 and the user interface 124 to increase the air pressure in the airways of the user 180 to aid in preventing the airways from closing and/or narrowing during sleep. The respiratory therapy device 122 can be positioned on a nightstand 188 that is directly adjacent to the bed 184 as shown in FIG. 1, or more generally, on any surface or structure that is generally adjacent to the bed 184 and/or the user 180.

The respiratory therapy system 120 can include or be, for example, a ventilator or a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Generally, a user who is prescribed usage of the respiratory therapy system 120 is someone who suffers from sleep apnea or other sleep related disorders. Such a user will tend to experience higher quality sleep and less fatigue during the day after using the respiratory therapy system 120 at night, compared to not using the respiratory therapy system 120. However, many users do not conform to their prescribed usage for a variety of reasons. In some cases, users do not conform to their prescribed usage because they wake up in the middle of the night (e.g., for a bathroom break, a water break, to feed a child, etc.) and simply forget to use their respiratory therapy systems when they fall back asleep. As such, the system 100 of the present disclosure can generate reminders to alert the user 180 to use the respiratory therapy system 120.

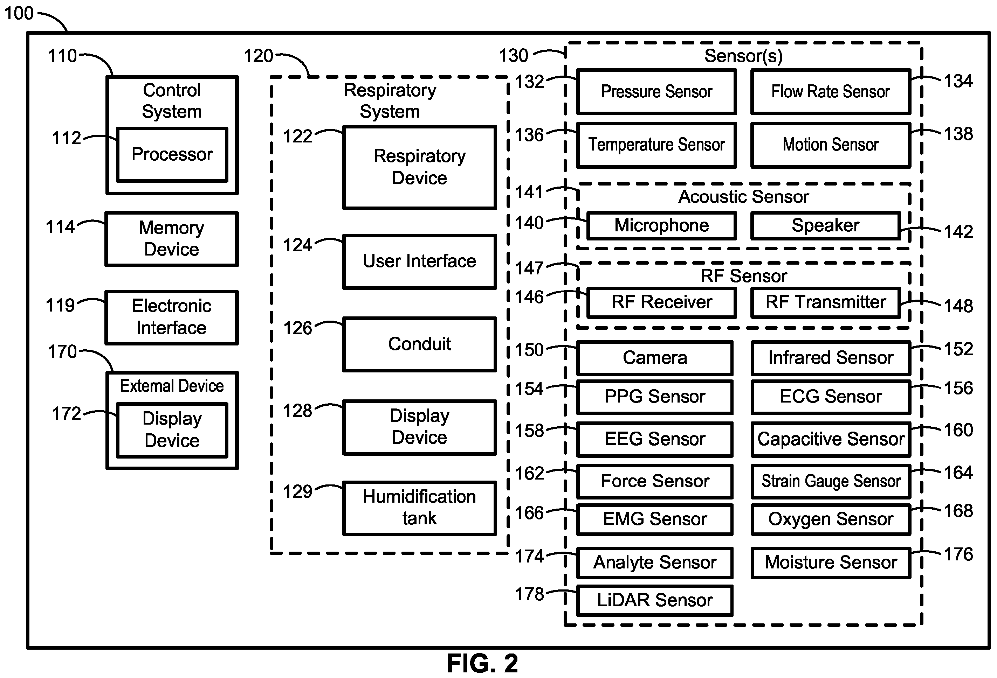

Referring to FIG. 2, the system 100 can include a control system 110, the respiratory therapy system 120, one or more sensors 130, and an external device 170. The system 100 can include the control system 110, a memory device 114, the display device 128, one or more sensors 130, and the humidification tank 129. Each of these components may or may not be part of the respiratory therapy device 122. As described herein, the system 100 generally can be used to generate data associated with a user (e.g., the user 180 of FIG. 1) during a plurality of sleep sessions and determine, based at least partially on the generated data, whether to generate an alarm reminding the user 180 to properly use the respiratory therapy system 120.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the external device 170, a portion (e.g., a housing) of the respiratory therapy system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122, within a housing of the external device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 (FIG. 1) stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a family history of insomnia, an employment status of the user, an educational status of the user, a socioeconomic status of the user, or any combination thereof. The medical information can include, for example, including indicative of one or more medical conditions associated with the user, medication usage by the user, or both. The medical information data can further include a multiple sleep latency test (MSLT) test result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or audio data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, over a cellular network, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the external device 170. In other implementations, the electronic interface 119 is coupled to or integrated (e.g., in a housing) with the control system 110 and/or the memory device 114.

The respiratory therapy system 120 (also referred to as a respiratory system) includes a respiratory therapy device 122 (also referred to herein as respiratory therapy device 122), a user interface 124, a conduit 126 (also referred to as a tube or an air circuit), a display device 128, and a humidification tank 129. In some implementations, the control system 110, the memory device 114, the display device 128, one or more of the sensors 130, and the humidification tank 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory therapy device 122, the user interface 124, the conduit 126, the display device 128, and the humidification tank 129, collectively as a system 120 (FIG. 1), can be used to treat individuals (e.g., a user) suffering from one or more sleep-related respiratory disorders (e.g., obstructive sleep apnea, central sleep apnea, or mixed sleep apnea).

The respiratory therapy device 122 is generally used to generate pressurized air that is delivered to a user, by way of the conduit 126 and the user interface 124. In some implementations, the respiratory therapy device 122 is configured to generate continuous constant air pressure that is delivered to the user. In other implementations, the respiratory therapy device 122 is configured to generate two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory therapy device 122 can also deliver pressurized air at a predetermined flow rate between, for example, about -20 L/min and about 150 L/min, while maintaining a positive pressure (relative to the ambient pressure).

The user interface 124 engages a portion of the user's face (see FIG. 1) and delivers pressurized air from the respiratory therapy device 122, via the conduit 126, to the user's airway to aid in preventing the airway from narrowing and/or collapsing during sleep. This may also increase the user's oxygen intake during sleep. As shown in FIG. 1, in some implementations, the user interface 124 is a facial mask that covers the nose and mouth of the user. Alternatively, the user interface 124 can be a nasal mask that provides air to the nose of the user or a nasal pillow mask that delivers air directly to the nostrils of the user. The user interface 124 can include a plurality of straps (e.g., including hook and loop fasteners) for positioning and/or stabilizing the user interface on a portion of the user (e.g., the face) and a conformal cushion (e.g., silicone, plastic, foam, etc.) that aids in providing an air-tight seal between the user interface 124 and the user 180. The user interface 124 can also include one or more vents for permitting the escape of carbon dioxide and other gases exhaled by the user 180. In other implementations, the user interface 124 includes a mouthpiece (e.g., a night guard mouthpiece molded to conform to the user's teeth, a mandibular repositioning device, etc.).

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep metric, a current date/time, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122 and includes a reservoir of water that can be used to humidify the pressurized air delivered from the respiratory therapy device 122. The respiratory therapy device 122 can include a heater to heat the water in the water reservoir 129, in order to humidify the pressurized air provided to the user. Additionally, in some implementations, the conduit 126 can also include a heating element (e.g., coupled to and/or imbedded in the conduit 126) that heats the pressurized air delivered to the user.

The one or more sensors 130 of the system 100 include a pressure sensor 132, a flow rate sensor 134, temperature sensor 136, a motion sensor 138, a microphone 140, a speaker 142, a radio-frequency (RF) receiver 146, an RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The data generated by one or more of the sensors 130 can be used by the control system 110 to determine a sleeping pattern of the user 180 for a plurality of sleep sessions. The sleeping pattern of the user 180 can include user behavior of the user 180, including a sleep routine of the user 180. The sleep routine of the user 180 can include the user 180 using a bathroom prior to falling asleep, the user 180 using a toothbrush prior to falling asleep, the user 180 relocating to a bedroom of the user 180 prior to falling asleep, the user 180 relocating to the bed 184 prior to falling asleep, the user 180 dimming lights of the bedroom prior to falling asleep, the user 180 turning off lights in the bedroom prior to falling asleep, the user 180 falling asleep at a certain time of day, or any combination thereof.

The data generated by one or more of the sensors 130 can be used by the control system 110 to determine a behavior pattern associated with the user 180 during a sleep session in a plurality of sleep sessions. For instance, the system described in WO 2015/006364 (see, for example, paragraphs [0169]-[0183]), can monitor the user's sleep patterns. A combination of flow and pressure sensors, used in the respiratory therapy device 122, may be used to detect events of taking off the user interface 124 during the night and putting it back on again. The behavior pattern associated with the user 180 includes one or more events including the user 180 waking up one or more times during a sleep session, the user 180 not engaging with the user interface 124 during a sleep session, the user 180 not engaging with the user interface 124 for a later portion of a sleep session, the user 180 having the user interface 124 disengaged for a length of time during a sleep session before re-engaging with the user interface 124, the user 180 experiencing one or more apnea events during a sleep session, prior to the user 180 disengaging with the user interface 124, the user 180 being in a specific sleep stage or a sequence of sleep stages during a sleep session, the user 180 having a regular waking time, or any combination thereof. A sleep stage can include wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), or any combination thereof.

The data generated by one or more of the sensors 130 can also be timestamped to determine a timeline associated with the sleeping pattern of the user 180 and/or the behavior pattern associated with the user 180. The data can be generated by one or more of the sensors 130 during a sleep session at a predetermined sampling rate, such as, for example, one sample per second, one sample per 30 seconds, one sample per minute, etc.

The pressure sensor 132 outputs pressure data that can be stored in the memory device 114 and/or analyzed by the processor 112 of the control system 110. In some implementations, the pressure sensor 132 is an air pressure sensor that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122. The pressure sensor 132 can be, for example, a capacitive sensor, an electromagnetic sensor, a piezoelectric sensor, a strain-gauge sensor, an optical sensor, a potentiometric sensor, or any combination thereof.

The flow rate sensor 134 outputs flow rate data that can be stored in the memory device 114 and/or analyzed by the processor 112 of the control system 110. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 180 (FIG. 1), a skin temperature of the user 180, a temperature of the air flowing from the respiratory therapy device 122 and/or through the conduit 126, in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The microphone 140 outputs sound data that can be stored in the memory device 114 and/or analyzed by the processor 112 of the control system 110. The microphone 140 can be used to record sound(s) during a sleep session (e.g., sounds from the user 180) to determine (e.g., using the control system 110) one or more sleep-related parameters, as described in further detail herein. The microphone 140 can be coupled to or integrated in the respiratory therapy device 122, the user interface 124, the conduit 126, or the external device 170.

The speaker 142 outputs sound waves that are audible to a user of the system 100 (e.g., the user 180 of FIG. 1). The speaker 142 can be used, for example, as an alarm clock, to play relaxing sounds, or to play an alert or message to the user 180 (e.g., in response to an event). The speaker 142 can be coupled to or integrated in the respiratory therapy device 122, the user interface 124, the conduit 126, or the external device 170.

The microphone 140 and the speaker 142 can be used as separate devices. In some implementations, the microphone 140 and the speaker 142 can be combined into an acoustic sensor 141, as described in, for example, WO 2018/050913 and WO 2020/104465 In such implementations, the speaker 142 generates or emits sound waves at a predetermined interval and/or frequency and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 180 or the bed partner 182 (FIG. 2). Based at least in part on the data from the microphone 140 and the speaker 142, the control system 110 can determine a location of the user 180 (FIG. 1) and/or one or more of the sleep-related parameters described in herein such as, for example, a respiration signal, a respiration rate, an inspiration amplitude, an expiration amplitude, an inspiration-expiration ratio, a number of events per hour, a pattern of events, a sleep state, pressure settings of the respiratory device 122, or any combination thereof. In this context, a sonar sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO2018/050913 and WO 2020/104465 mentioned above.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 146, and this data can be analyzed by the control system 110 to determine a location of the user 180 (FIG. 1) and/or one or more of the sleep-related parameters described herein. An RF receiver (either the RF receiver 146 and RF transmitter 148) or another RF pair), can also be used for wireless communication between the control system 110, the respiratory therapy device 122, the one or more sensors 130, the external device 170, or any combination thereof. While the RF receiver 146 and RF transmitter 148 are shown as being separate and distinct elements in FIG. 2, in some implementations, the RF receiver 146 and RF transmitter 148 are combined as a part of an RF sensor 147. In some implementations, the RF sensor 147 includes a control circuit. The specific format of the RF communication could be WiFi, Bluetooth, etc.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify the location of the user, determine a time when the user 180 enters the bed 184 and a time when the user 180 exits the bed 184.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 180 and/or movement of the user 180. The IR sensor 152 can also be used in conjunction with the camera 150 when measuring the presence, location, and/or movement of the user 180. The IR sensor 152 can detect infrared light having a wavelength between about 700 nm and about 1 mm, for example, while the camera 150 can detect visible light having a wavelength between about 380 nm and about 740 nm.

The PPG sensor 154 outputs data associated with the user 180 (FIG. 1) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 180 and/or embedded in clothing and/or fabric that is worn by the user 180, in the user interface 124 and/or its associated headgear (e.g., straps, etc.).

The ECG sensor 156 outputs data associated with electrical activity of the heart of the user 180. In some implementations, the ECG sensor 156 includes one or more electrodes that are positioned on or around a portion of the user 180 during a sleep session. The physiological data from the ECG sensor 156 can be used, for example, to determine one or more of the sleep-related parameters described herein.

The EEG sensor 158 outputs data associated with electrical activity of the brain of the user 180. In some implementations, the EEG sensor 158 includes one or more electrodes that are positioned on or around the scalp of the user 180 during a sleep session. The data from the EEG sensor 158 can be used, for example, to determine a sleep state of the user 180 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs data associated with electrical activity produced by one or more muscles. In some implementations, the one or more sensors 130 also include a galvanic skin response (GSR) sensor, a blood flow sensor, a respiration sensor, a pulse sensor, a sphygmomanometer sensor, an oximetry sensor, an oxygen sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory therapy device 122, etc.). Thus, in some implementations, the moisture sensor 176 can be coupled to or integrated in the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 2, any combination of the one or more sensors 130 can be integrated in and/or coupled to any one or more of the components of the system 100, including the respiratory therapy device 122, the user interface 124, the conduit 126, the humidification tank 129, the control system 110, the external device 170, or any combination thereof. For example, the microphone 140 and the speaker 142 can be integrated in and/or coupled to the external device 170. In such implementations, the external device 170 can be considered a secondary device that generates additional or secondary data for use by the system 100 (e.g., the control system 110) according to the aspects of the present disclosure. In some implementations, at least one of the one or more sensors 130 is not coupled to the respiratory therapy device 122, the control system 110, or the external device 170, and is positioned generally adjacent to the user 180 during the sleep session (e.g., positioned on or in contact with a portion of the user 180, worn by the user 180, coupled to or positioned on the nightstand 188, coupled to the mattress 186, coupled to the ceiling, etc.).

The external device 170 (FIG. 2) includes a display device 172. The external device 170 can be, for example, a mobile device such as a smart phone, a tablet, a laptop, or the like. Alternatively, the external device 170 can be an external sensing system, a television (e.g., a smart television) or another smart home device (e.g., smart speaker(s)). In some implementations, the external device 170 is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the external device 170. In some implementations, one or more external devices can be used by and/or included in the system 100.

While the control system 110 and the memory device 114 are described and shown in FIG. 2 as being a separate and distinct component of the system 100, in some implementations, the control system 110 and/or the memory device 114 are integrated in the external device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc.), or any combination thereof.

Each of the elements in the system 100 can be positioned in one or more housings and in one or more physical locations such that any combination or portion thereof of the elements shown in the system 100 in FIG. 2 can be in a first housing and any other combination or portion thereof of the elements shown in the system 100 in FIG. 2 can be in a second housing that is separate and distinct from the first housing, etc. For example, in some implementations, the respiratory therapy device 122 includes a first housing and the control system 110, the memory device 114, and one or more of the sensors 130 are all included the first housing. For another example, in some implementations, the respiratory therapy device 122 includes a first housing and the control system 110, the memory device 114, and a first one of the sensors 130 are all included the first housing and a second one of the sensors 130 is in a second housing. For another example, in some implementations, the respiratory therapy device 122 includes a first housing and one or more of the sensors 130 is included the first housing and the control system 110 and the memory device 114 are in a second housing that is remote from the first housing.

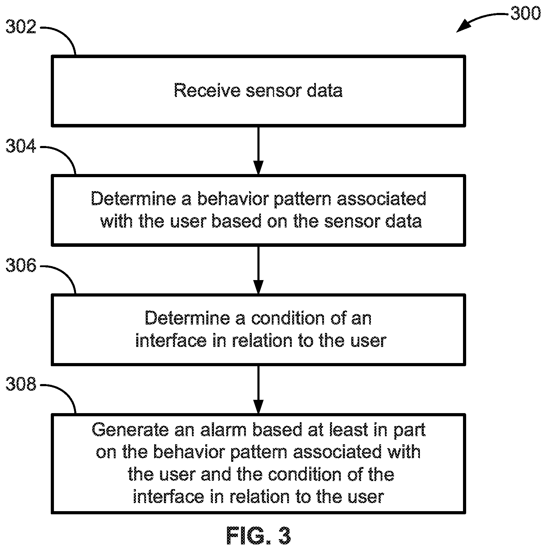

Referring to FIG. 3, a method 300 for generating an alarm based at least in part on data associated with the user 180 is illustrated according to some implementations of the present disclosure. One or more of the steps of the method 300 described herein can be implemented using the system 100 (FIG. 2).

At step 302, the control system 110 receives data from the one or more sensors 130 as already described herein in connection with FIG. 2. The received data can be obtained from the respiratory therapy device 122 generating pressure data via the pressure sensor 132. The pressure data generated can be used to determine other data associated with the user 180 including a respiration rate of the user 180, apnea events experienced by the user 180, etc. The received data can be generated by the microphone 140 generating sound data while the user 180 is awake, while the user 180 is trying to fall asleep, or while the user 180 is asleep. The received data can be generated by the microphone 140 and the speaker 142 acting as an acoustic sensor as described herein for generating movement data that is indicative of movement of the user 180. The received data can be generated by the RF transmitter 148 and the RF receiver 146 acting as a radar sensor to generate movement data indicating movement of the user 180.

The data generated by the one or more sensors 130 that is received by the control system 110 can include the respiration rate of the user 180, a breath analysis of the user 180, an indicator of the airway resistance of the user 180, blood flow to the brain of the user 180, blood pressure of the user 180, skin temperature of the user 180, core temperature of the user 180, brain activity of the user 180, heart rate of the user 180, muscle tone of the user 180, sympathetic nerve activity of the user 180, an activity level of the user 180 (e.g., body movement, chest movement, limb movement, body position, coughing in bed, etc.), blood oxygen saturation (SpO2) of the user 180, expired carbon dioxide (CO₂) of the user 180, or any combination thereof.

The data generated by the one or more sensors 130 can be data associated with a plurality of sleep sessions. A sleep session can be defined in multiple ways. In some implementations, a sleep session is a duration where the user 180 is asleep, that is, the sleep session has a start time and an end time, and during the sleep session, the user 180 does not wake until after the end time. That is, any period of the user 180 being awake is not included in a sleep session. From this first definition of sleep session, if the user 180 wakes up and falls asleep multiple times in a same night, each of the sleep intervals separated by an awake interval is a sleep session.

Alternatively, in some implementations, a sleep session has a start time and an end time, and during the sleep session, the user 180 can wake up so long as a continuous duration that the user 180 is awake is below an awake duration threshold. The awake duration threshold can be defined as a percentage of a sleep session. The awake duration threshold can be, for example, ten percent of the sleep session duration, five percent of the sleep session duration, two percent of the sleep session duration, etc. In some implementations, the awake duration threshold is defined as a fixed amount, for example, thirty minutes, fifteen minutes, ten minutes, five minutes, etc.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

At step 304, the control system 110 analyzes the received data from step 302 to determine a behavior pattern associated with the user 180. The behavior pattern may be based on the data measured during the last sleep session alone. In some implementations, the received data includes historical data associated with one or more prior sleep sessions and current data associated with a current sleep session. The one or more prior sleep sessions and the current sleep session constitute the plurality of sleep sessions. The plurality of sleep sessions may be all included in a single night, or cover a plurality of nights. The historical data can be windowed such that only data associated with a predetermined number of more recent sleep sessions are considered. For example, the historical data includes data associated with sleep sessions of a past week. In another example, the historical data includes data associated with a set number of the prior sleep sessions (e.g., five prior sleep sessions, ten prior sleep sessions, twenty prior sleep sessions, etc.). The quality of the data may also be included as additional criteria for whether data from a specific sleep session should be included under consideration.

The control system 110 analyzes the historical data to determine a behavior pattern associated with the user 180 for the one or more prior sleep sessions. The behavior pattern can include one or more events as previously discussed in connection with FIG. 2. For example, the behavior pattern can include the user 180 not engaging with the user interface 124 during at least one of the prior sleep sessions, the user 180 disengaging with the user interface 124 and not re-engaging for a later portion of at least one of the prior sleep sessions, etc. The behavior pattern of the user 180 can describe a habit pattern of the user 180 in relation to how the user 180 uses the respiratory therapy device 122. That is, the behavior pattern associated with the user 180 describes prior sleeping patterns of the user 180 and prior usage patterns of the respiratory therapy device 122 by the user 180.

In some implementations, the control system 110 can determine whether the user 180 wakes up one or more times during the one or more prior sleep sessions by analyzing motion data obtained from the motion sensor 138. If the user 180 moved from one location in her living area to another, the control system 110 can determine that the user 180 was awake during the move. Instead of, or in addition to, the above, the control system 110 can determine whether the user 180 wakes up one or more times during the one or more prior sleep sessions by analyzing sound data obtained from the microphone 140. The sound data can be analyzed to determine breath patterns for distinguishing times when the user 180 is asleep and times when the user 180 is awake, during the one or more prior sleep sessions. Such sound data can be used to complement the data received from the motion sensor 138, to, for example, improve the accuracy of the determination. Similarly, the control system 110 can use any of the generated data from others of the one or more sensors 130 to distinguish between the user 180 being asleep or awake in the one or more prior sleep sessions. Accordingly, from asleep-awake cycles extracted from received data associated with the one or more prior sleep sessions, the control system 110 can approximate a typical bedtime for the user 180 in the evening, a typical wake-up time for the user 180 in the morning, as well as one or more additional waking up times and/or falling-asleep times throughout the night.

In some implementations, the control system 110 can analyze the received data to determine that the user 180 did not engage with the user interface 124 during one or more of the prior sleep sessions. For example, when the user 180 is not engaged with the user interface 124, the respiratory therapy device 122 discontinues supply of the pressurized air to the user interface 124. The control system 110 can determine timestamps and durations when the respiratory therapy device 122 discontinues supplying the pressurized air to the user interface 124 during the one or more prior sleep sessions. The control system 110 can also determine whether the user 180 was asleep while not engaging with the user interface 124 (e.g., via analysis of breathing patterns determined with sound data obtained from the microphone 140 or with the sleep stage measurement capabilities of the RF sensor 147 (see e.g., WO 2015/006364)).

In some implementations, the control system 110 can determine other behavior patterns during the one or more prior sleep sessions. For example, the control system 110 can determine that the user 180 did not engage with the user interface 124 for a later portion of at least one of the one or more prior sleep sessions. In another example, the control system 110 can determine that the user 180 disengaged with the user interface 124 for a measured length of time before re-engaging with the user interface 124 again during at least one of the one or more prior sleep sessions.

In some implementations, the control system 110 can determine whether the user 180 was in a specific sleep stage or a sequence of sleep stages during one of the one or more prior sleep sessions prior to the user 180 disengaging the user interface 124 and not engaging with the user interface 124 for a later portion of at least one of the one or more prior sleep sessions. In some implementations, the control system 110 can determine whether the user 180 was in a specific sleep stage or a combination of sleep stages during one of the one or more prior sleep sessions prior to the user 180 disengaging the user interface 124 for a length of time before re-engaging with the user interface 124 during the one of the one or more prior sleep sessions. A possible link between a specific sleep stage or a sequence of sleep stages with the actions of the user upon waking up, may be established.

In some implementations, the control system 110 can analyze the historical data to determine that the user 180 experienced apnea events during the one or more prior sleep sessions. The control system 110 can determine whether the apnea events are correlated with the user not having a user interface 124 as a result of the user 180 waking up and disengaging with the user interface 124.

At step 306, the control system 110 determines a condition of the user interface 124 in relation to the user 180. The control system 110 analyzes current data associated with the current sleep session to determine the condition of the user interface 124 in relation to the user 180. For example, the user 180 can awake in the current sleep session and disengage from the user interface 124. In some implementations, the control system 110 can determine from the current data that the user interface 124 has been disengaged from the user 180. In some implementations, the control system 110 can determine that the user interface 124 is not securely engaged to the user 180 and that the user interface 124 is leaking pressurized air.

In some implementations, the control system 110 determines the condition of the user interface 124 by causing the respiratory therapy device 122 to pulse the supplied pressurized air. The flow rate sensor 134 and/or the pressure sensor 132 can be used to measure air resistance and/or pressure in the conduit 126 and/or the user interface 124. If the air resistance and/or pressure is below a threshold, then the control system 110 determines that the user interface 124 is not securely engaged to the user 180.

In some implementations, the control system 110 determines the condition of the user interface 124 by detecting an abrupt increase in intentional leak. For example, when the user 180 removes the user interface 124, the pressurized air that should be supplied to the user 180 will instead be lost to the environment. In some implementations, the user interface 124 moves or slips while the user 180 changes position. The movement of the user interface 124 can cause an abrupt increase in leakage from the user interface 124.

At step 308, the control system 110 generates an alarm based at least in part on the behavior pattern associated with the user 180 and the condition of the user interface 124 in relation to the user 180. In some implementations, the control system 110 generates the alarm based on the user interface 124 being disengaged from the user 180. In other implementations, the control system 110 generates the alarm based on the user interface 124 being disengaged from the user 180 for a predetermined amount of time. The predetermined amount of time can be a predetermined fixed amount of time, or be determined on a night by night basis, or on a sleep session by sleep session basis, based on the historical data and/or the behavior pattern associated with the user 180. For example, the control system 110 can determine from at least one of the prior sleep sessions that the user 180 usually wakes up for ten minutes during a sleep session (e.g., to feed a baby, etc.). The control system 110 can monitor the user interface 124 and the respiratory therapy system 120 to determine whether the user 180 has re-engaged with the user interface 124 within ten minutes. If the user 180 has not re-engaged with the user interface 124 within ten minutes, then the control system 110 can generate the alarm.

In some implementations, the control system 110 generates the alarm based at least in part on a type of event in the behavior pattern associated with the user 180, a frequency of the event in the behavior pattern, a time of night of occurrence of the event in the behavior pattern, or any combination thereof. For example, the control system 110 can determine at step 304 that the user 180 has a regular waking time in the morning, and as such when the user 180 disengages from the user interface 124 around this regular waking time, the control system 110 does not generate the alarm. In another example, the control system 110 can determine at step 304 that the user 180 has a regular waking time at night, after which the user returns to bed, and when the user 180 disengages from the user interface 124 around that time, the control system 110 can determine that the user 180 is expected to go back to sleep. The control system 110 can generate the alarm to remind the user 180 to don the user interface 124 before going back to sleep. In some implementations, a length of the remaining duration of the current sleep session is taken into account on whether to generate the alarm. For example, if the user 180 is expected to wake up in twenty minutes, then the control system 110 does not generate the alarm. On the other hand, if the user 180 is expected to wake up in forty-five minutes or more, then the control system 110 generates the alarm. In some implementations, a duration of therapy received by the user 180 is taken into account. For example, if the user 180 has received a minimum duration of therapy (e.g. 2, 3 4 or 5 hours etc.) during a sleep session before the user disengages from the user interface 124, then the control system 110 does not generate the alarm. Additionally, or alternatively, if the number of events experienced by a user during a sleep session, when on and/or off therapy, was maintained below a threshold, optionally for a minimum duration of time (e.g. 2, 3 4 or 5 hours etc.) during a sleep session, then the control system 110 does not generate the alarm.

In some implementations, the control system 110 generates the alarm based on the condition of the user interface 124 indicating that the user interface 124 is not securely engaged to the user 180 and a remaining duration of the current sleep session is greater than a sleep duration threshold. For example, the user 180 may engage with the user interface 124 in a manner that supplied pressurized air leaks from the user interface 124. The leakage discussed here does not refer to the standard airflow through the exhaust vents of (e.g., intentional leak) the user interface 124, but to a leak caused by a less than optimal seal of the user interface 124 with the patient's airways (e.g., unintentional leak). The respiratory therapy device 122 can include one or more algorithms that can detect the presence of leaks such as intentional (e.g., vent leak) or unintentional leaks (e.g., mask leak, mouth leak, etc.). The microphone 140 and/or the pressure sensor 132 can also be used to determine excessive leakage in the user interface 124. If the noise coming from the user interface 124 exceeds a noise threshold (loudness and/or duration, etc.), the control system 110 can determine that the user interface 124 has excessive leakage. If a differential pressure between the user interface 124 and the respiratory therapy device 122 is above a threshold, then the control system 110 can determine that the user interface 124 has excessive leakage. When the user 180 sleeps with the user interface 124 not securely engaged, the control system 110 can estimate the remaining duration of the current sleep session based on the behavior pattern determined at step 304. If the remaining duration is above the sleep duration threshold, then the control system 110 generates the alarm. The sleep duration threshold can be forty-five minutes, an hour, etc.

In some implementations, the sleep duration threshold is determined based on a likelihood that the user 180 will experience an apnea event during the remaining duration of the current sleep session. The likelihood that the user 180 will experience an apnea event can be calculated from the historical data as a number of apnea events suffered by the user 180 per unit time when the user interface 124 is not securely engaged to the user 180 multiplied by the remaining duration of the current sleep session. The determined likelihood can be compared against a threshold to determine whether to generate the alarm. For example, if the determined likelihood is greater than 0.90, then the control system generates the alarm.

In some implementations, the control system 110 generates the alarm based on the condition of the user interface 124 indicating that the user interface 124 is not securely engaged to the user 180 and the user 180 historically does not sleep well, either in general, or with the user interface 124 sub-optimally engaged. For example, the control system 110 calculates a historical sleep metric associated with the user 180. The historical sleep metric can be determined as an average number of times the user 180 woke up during the one or more prior sleep sessions. The historical sleep metric can be compared to a historical score threshold, and when above the historical score threshold, then the control system 110 generates the alarm. The control system 110 makes a determination that the user 180 usually does not sleep well because of not securely engaging with the user interface 124, thus when multiple prior sleep sessions presents a pattern of interruption of the sleep of the user 180, then the control system 110 generates the alarm to alert the user 180 of the improper engagement with the user interface 124.

In some implementations, the control system 110 generates the alarm based on the condition of the user interface 124 indicating that the user interface 124 is not securely engaged to the user 180 and the user 180 is not currently sleeping well. For example, the control system 110 calculates a current sleep metric associated with the user 180. The current sleep metric can be determined as an average number of times the user 180 has experienced an apnea event during the current sleep session, an average number of times the user 180 is moving during the current sleep session, an average number of times the user 180 has woken up during the current sleep session, a length of time that the user spends in specific sleep stages during the current sleep session, or any combination thereof. The current sleep metric can be determined, using the sleep staging capabilities of the RF sensor 147 and/or the acoustic sensor 141, or based on a breathing pattern of the user 180, a blood pressure of the user 180, a heart rate of the user 180, a body movement of the user 180, a chest movement of the user 180, a limb movement of the user 180, a body position of the user 180, or any combination thereof. When the current sleep metric is above a current score threshold, then the control system 110 generates the alarm to alert the user 180 to securely engage with the user interface 124.

The alarm generated can be an audible alarm, a visual alarm, a vibratory alarm, a notification displayed on the external device 170, or any combination thereof. In some implementation, the control system 110 causes the respiratory therapy device 122 to generate the alarm. The respiratory therapy device 122 can generate the alarm by pulsing a flow of the supplied pressurized air for a period of time such that the user interface 124 emits a sound during the period of time. The sound emitted by the respiratory therapy device 122 via the user interface 124 can be emitted intermittently. The period of time when the sound is emitted can be between two seconds and thirty seconds. In some implementations, the period is ten seconds. In some implementations when the user interface 124 is not properly secured to the user 180 and is excessively leaking, pulsing a flow of the supplied pressurized air provides haptic feedback to the user 180 to cause a minor discomfort for the user 180 to get up and securely engage with the user interface 124. Pulsing the flow of the supplied pressurized air in this manner can reduce a risk of waking the bed partner 182.

In some implementation, the one or more light sources are located on, or otherwise integrated in, the user interface 124 and/or conduit 126. Suitable light sources include optical fibers, light emitting diodes, etc. In other implementation, the one or more light sources located away from the user interface 124 and/or conduit 126 and are configured to direct light onto and illuminate the user interface 124 and/or conduit 126. The one or more light sources can become activated when the user removes the user interface 124, such as when taking a bathroom break during a therapy session. Alternatively, the one or more light sources can become activated when the user removes the user interface 124 and only once the user is detected returning to the bedroom environment, using, for example, motion sensors (such as a LiDAR sensor, PIR sensor, etc.) to detect the exit and/or entrance of the user to the bedroom environment. By activating the one or more light sources only after the user returns to the bedroom environment, disturbance of the bed partner and/or power use is minimized while allowing the user to readily locate and re-don the user interface 124. In implementations, if the presence of a bed partner is detected, such as via RF sensor 147 and/or the acoustic sensor 141, the intensity of the light from the one or more light sources may be reduced relative to a standard operating level in order to minimize impact on the bed partner. In some implementations, the sleep state or sleep stage of the user can be monitored to detect or predict an awakening of the user and the one or more lights sources may be activated accordingly. Additionally, or alternatively, the one or more light sources may be manually activated by the user when he/she awakens. To assist with this, such as in a darkened room, a button/switch to activate (and/or turn off) the one or more light sources may be illuminated. Similar to described previously, the sleep state or sleep stage of the user can be monitored to detect or predict an awakening of the user and the button/switch may be illuminated accordingly.

In some implementations, the control system 110 causes the respiratory therapy system 120 to generate the alarm by lighting up the display device 128. The respiratory therapy system 120 can also generate the alarm by lighting up one or more light sources present in the user interface 124 and/or the conduit 126. As such, the user 180 can see where the user interface 124 is located in a dark room for ease of donning the user interface 124. In some implementations, different colors of light can be cycled. For example, red light can transition to green light or can cycle between red and green until the user interface 124 is securely engaged to the user 180. Generating light as the alarm reduces a risk of waking the bed partner 182.

In some implementations, the control system 110 causes the speaker 142 to generate the alarm. The speaker 142 generates the alarm by emitting a sound. The sound emitted from the speaker can include relaxing sounds like ocean sounds, waves breaking, waterfall, white noise, shaped noise, or any combination thereof. The relaxing sounds may not only remind the user 180 to properly secure the user interface 124 but can also be used as a sleep aid to allow the user 180 to fall asleep after securing the user interface 124. In some implementations, instead of having a separate speaker, a speaker on the respiratory therapy device 122 can provide alarm melodies, tunes, or sounds. In some implementations, the alarm can be sounded at progressively increasing frequency and volume.

In some implementations, the control system 110 generates the alarm by sending a notification to the external device 170 (e.g., a mobile device associated with the user 180). The external device 170 can provide the notification to the user 180 by vibrating a housing of the external device 170, lighting up the display device 172, making a sound from speakers provided on the external device 170, displaying a message on the display device 172, etc. In some implementations, the external device 170 is a smart pillow that vibrates to provide haptic feedback to remind the user 180 to securely engage the user interface 124.

In some implementations, the control system 110 determines that the user 180 has interacted with the external device 170, and based on this interaction, generates the alarm by sending a notification to the external device 170. For example, the user 180 wakes up, disengages the user interface 124, and later interacts with her mobile phone. The camera 150 can capture light from the mobile phone indicating the mobile phone being in use, the speaker 142 can capture sounds from the mobile phone indicating the mobile phone being in use, the mobile phone can send a signal to the control system 110 indicating that a touchscreen of the mobile phone is receiving inputs, and/or the mobile phone can send a signal to the control system 110 indicating that an accelerometer and/or gyroscope of the mobile phone senses the mobile phone is moving. Since the user 180 is engaging the mobile phone, the control system 110 can send a notification to the mobile phone to alert the user 180 to use the user interface 124. The notification can be a push notification.

In some implementations, for example, when it is detected that the user uses the external device 170, the control system 110 sends a notification to the external device 170 (e.g., a mobile phone of the user 180) for instructing the user 180 to reduce pressure of the supplied pressurized air in order to reduce leakage in the user interface 124 and promote a more comfortable and secure fit of the user interface 124. In some implementations, the control system 110 causes the respiratory therapy device 122 to reduce the pressure of the supplied pressurized air when the user interface 124 leaks.

In some implementations, prior to generating the alarm, the control system 110 sets a nature of the alarm. The nature of the alarm can include a type of sound to be produced for alerting the user 180, a type of vibration to be produced for alerting the user 180, a type of notification to be produced for alerting the user 180, a type of light to be produced for alerting the user 180, or any combination thereof.

The type of sound to be produced can include a sound with constant amplitude, a sound with constant frequency, an intermittent sound, a sound from a speaker, a sound produced by pressurized air, a sound produced by vibration of the external device 170, a sound produced by vibration of a motor of the respiratory therapy device 122, or any combination thereof.

The type of vibration to be produced for alerting the user 180 can include a vibration caused by varying pressure of the pressurized air provided by the respiratory therapy device 122, a vibration caused by the external device 170, a vibration caused by a motor of the respiratory therapy device 122, or any combination thereof.

The type of notification to be produced for alerting the user 180 can include displaying a message on the display device 172, displaying a message on the display device 128, or any combination thereof. The type of light to be produced for alerting the user 180 can include flashing of the display device 172, flashing of the display device 128, lighting up the respiratory therapy system 120, a color of light to be produced, as described herein, or any combination thereof.

In some implementations, prior to generating the alarm at step 308, the control system 110 sets a magnitude of the alarm. The magnitude of the alarm can include a volume for a sound alarm, the time modulation of the volume for the sound alarm (e.g., increasing volume over time, decreasing volume over time, a rate of decreasing and/or increasing volume, etc.), an intensity for a vibration alarm, a shape of the intensity for the vibration alarm (e.g., increasing vigor of vibration over time, decreasing vigor of vibration over time, a rate of decreasing and/or increasing the vibration, etc.), an intensity for a light alarm, the intensity modulation for the light alarm (e.g., increasing the intensity for the light alarm over time, decreasing the intensity for the light alarm over time, a rate of increasing and/or decreasing the light alarm, etc.), or any combination thereof. In the case of a sound alarm, apart from amplitude modulation, the modulation of the alarm may also include variation of the sound frequency. For example, a predetermined frequency range may be scanned during several initial sleep sessions to identify the user's sensitivity to different frequencies. The alarms provided to the user in future sessions may attempt to target the most impactful frequencies (e.g., in the case of a heavy sleeper) or the less impactful frequencies (e.g., in the case of a light sleeper).

In some implementations, prior to generating the alarm at step 308, the control system 110 sets a duration for the alarm. The control system 110 can set the duration of the alarm between two seconds and thirty seconds. In some implementations, the set duration for the alarm is variable, such that the set duration can be changed over time.

In some implementations, prior to generating the alarm at step 308, the control system 110 sets a period for re-triggering the alarm. Once the alarm is generated without any response from the user 180 within the set duration for the alarm, the period for re-triggering the alarm is a waiting period before the control system 110 can re-trigger the alarm for the set duration. In some implementations, the set period for re-triggering the alarm can be variable, such that the set period for re-triggering the alarm can be increased over time or decreased over time.

In some implementations, the control system 110 can determine, for example, during the time of the alarm or during a waiting period, that the condition of the user interface 124 changes from the user interface 124 not being securely engaged to the user 180 to the user interface 124 being securely engaged to the user 180. The user 180 may have responded to the alarm generated at step 308 and correctly donned the user interface 124, thus the control system 110 senses the change in the condition of the user interface 124. Since the user 180 is awake and recently donned the user interface 124, the control system 110 causes the respiratory therapy device 122 to provide the supplied pressurized air at a relatively lower pressure than a prescribed treatment pressure for the user 180. Over time, the control system 110 can cause the respiratory therapy device 122 to ramp up the pressure of the supplied pressurized air from the relatively lower pressure to the prescribed treatment pressure over a predetermined amount of time. Ramping from the relatively lower pressure to the prescribed treatment pressure increases user comfort while using the respiratory therapy system 120. In some implementations, after detecting the donning the user interface 124, the control system 110 stops the generated alarm.

In some implementations, the control system 110 can generate a report for the current sleep session. The report can include a number of alarms generated during the current sleep session. The report can further include a nature of each of the alarms generated. The report can further include a response of the user 180 to each of the alarms generated. The response of the user 180 can include the user 180 dismissing one or more of the generated alarms, the user 180 ignoring one or more of the generated alarms until the one or more generated alarms time out, the user 180 re-engaging or engaging with the user interface 124, or any combination thereof. Here the term dismissing indicates that the user actively stops the alarm, whilst the term ignoring indicates that the system did not record any active response from the user (e.g., the user neither donned the user interface 124, nor dismissed the alarm).

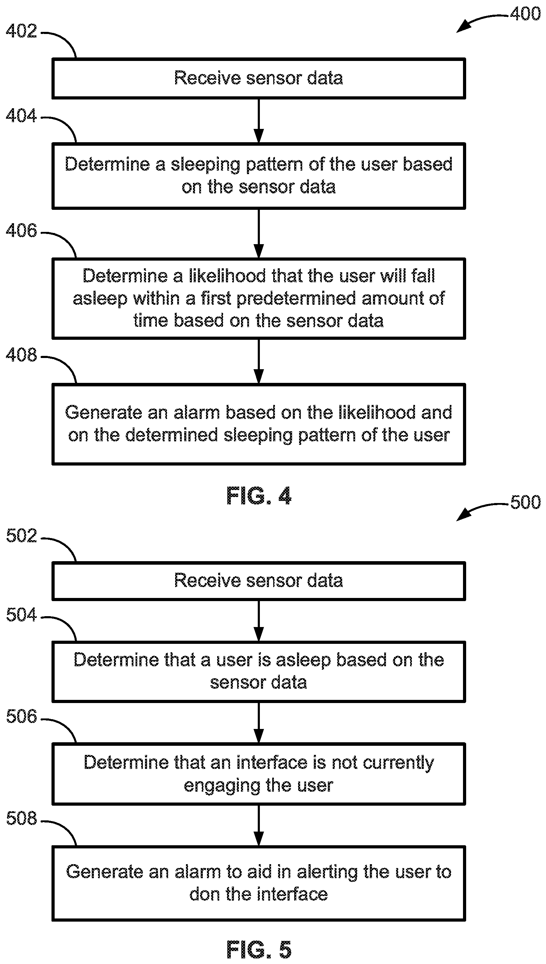

Referring to FIG. 4, a method 400 for generating an alarm based at least in part on a likelihood that the user 180 of the respiratory therapy device 122 will fall asleep within a predetermined amount of time is provided, according to some implementations of the present disclosure. One or more of the steps of the method 400 described herein can be implemented using the system 100 (FIG. 2). At step 402, the control system 110 receives data from the one or more sensors 130. Step 402 is analogous to step 302 as already described in connection with FIG. 3.

At step 404, the control system 110 analyzes the received data from step 402 to determine a sleeping pattern of the user 180. In some implementations, the received data include historical data associated with one or more prior sleep sessions and current data being generated by the one or more sensors 130. The one or more prior sleep sessions can be windowed as previously discussed in connection with step 304 of FIG. 3. The current data being generated by the one or more sensors 130 can include movement data, a time of day or other data as previously described in connection with the one or more sensors 130.

The historical data is analyzed to determine a sleeping pattern of the user 180. The sleeping pattern of the user 180 can include user behavior and circumstances that occurred in previous sleep sessions. User behavior can include a sleep routine of the user 180. The sleep routine of the user 180 can include the user 180 using a bathroom prior to falling asleep, the user 180 using a toothbrush prior to falling asleep, the user 180 relocating to a bedroom of the user 180 prior to falling asleep, the user 180 relocating to the bed 184 prior to falling asleep, the user 180 dimming lights of the bedroom prior to falling asleep, the user 180 turning off lights in the bedroom prior to falling asleep, the user 180 falling asleep at a certain time of day, or any combination thereof.

At step 406, the control system 110 determines, based at least in part on the current data and the determined sleeping pattern, a likelihood that the user 180 will fall asleep within a first predetermined amount of time. In some implementations, the first predetermined amount of time can be ten minutes or any time between two minutes to fifteen minutes. In some implementations, the likelihood that the user will fall asleep is determined via a machine learning algorithm taking as inputs the current data and a current time of day. That is, based on the historical data and the sleeping pattern of the user 180, the control system 110 can determine the likelihood based on an average amount of time that the user 180 falls asleep after one or more events is identified under the sleeping patterns determined at step 404. In some implementations, the likelihood can be determined based on state machines where identified sleeping patterns of the user 180 are plotted in a state space, and each state in the state space has an associated lead time for the user 180 falling asleep. The likelihood can be determined as the first predetermined amount of time divided by the lead time and normalized to a value between 0 and 1.

At step 408, the control system 110 generates an alarm based at least in part on the determined likelihood and on the determined sleeping pattern of the user 180. The control system 110 can generate the alarm to remind the user 180 to don the user interface 124 prior to going to sleep if the determined likelihood is above a threshold (e.g., 0.9). The alarm generated can be of various forms as described in connection with step 308 of FIG. 3. In some implementations, the control system 110 can generate the alarm based on a condition of the user interface 124 in relation to the user 180. That is, if the user 180 is already engaged with the user interface 124, the control system 110 does not generate the alarm.

Referring to FIG. 5, a method 500 for generating an alarm based at least in part on data associated with the user 180 of the respiratory therapy device 122 is provided, according to some implementations of the present disclosure. One or more of the steps of the method 500 described herein can be implemented using the system 100 (FIG. 2). At step 502, the control system 110 receives data from the one or more sensors 130. Step 502 is analogous to step 302 as already described in connection with FIG. 3.

At step 504, the control system 110 determines, based at least in part on the received data, that the user 180 is currently asleep. In some implementations, the control system 110 determines that the user 180 is asleep based on a breathing pattern of the user 180 obtained via the microphone 140. In some implementations, the control system 110 determines that the user 180 is asleep based on limb movements, chest movements, or body movements of the user 180 (e.g., as detected by the RF sensor 147 and/or the acoustic sensor 141).

At step 506, the control system 110 determines, based at least in part on the received data, that the user interface 124 is not currently engaging the user 180. The control system 110 can determine that the user interface 124 is not currently engaging the user 180 in ways similar to those described in connection with step 306 of FIG. 3.

At step 508, the control system 110 generates the alarm to aid in alerting the user 180 to don the user interface 124. Step 508 can be similar to or analogous to step 308 as described herein in connection with FIG. 3. In some implementations, the control system 110 can generate the alarm based on historical sleep metrics and/or current sleep metrics as previously described in connection with FIG. 3.

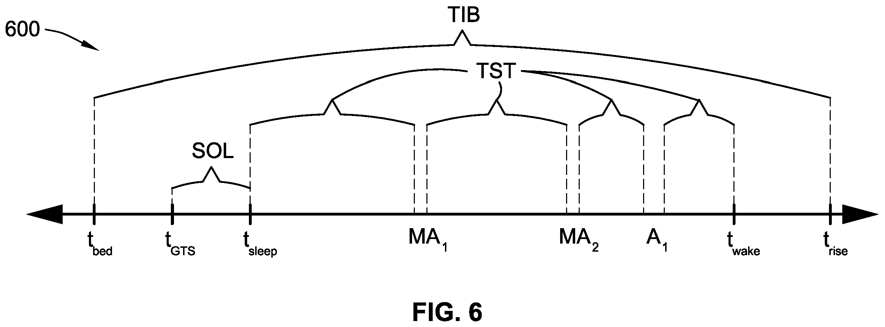

As used herein, a sleep session can be defined in a number of ways based on, for example, an initial start time and an end time. Referring to FIG. 6, an exemplary timeline 600 for a sleep session is illustrated. The timeline 600 includes an enter bed time (t_{bed}), a go-to-sleep time (t_{GTS}), an initial sleep time (tₛₗₑₑₚ), a first micro-awakening MA₁ and a second micro-awakening MA₂, a wake-up time (t_{wake}), and a rising time (tᵣᵢₛₑ).

As used herein, a sleep session can be defined in multiple ways. For example, a sleep session can be defined by an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, September 7, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, September 8, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the external device 170 (FIG. 1) to manually initiate or terminate the sleep session.

Referring to FIG. 6, the enter bed time t_{bed} is associated with the time that the user initially enters the bed (e.g., the bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the external device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (T_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 600 of FIG. 6, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (T_{GTS}) and ending at the wake-up time (t_{wake}). In some implementations, a sleep session is defined as starting at the go-to-sleep time (t_{GTS}) and ending at the rising time (tᵣᵢₛₑ). In some implementations, a sleep session is defined as starting at the enter bed time (t_{bea}) and ending at the wake-up time (t_{wake}). In some implementations, a sleep session is defined as starting at the initial sleep time (tₛₗₑₑₚ) and ending at the rising time (tᵣᵢₛₑ).

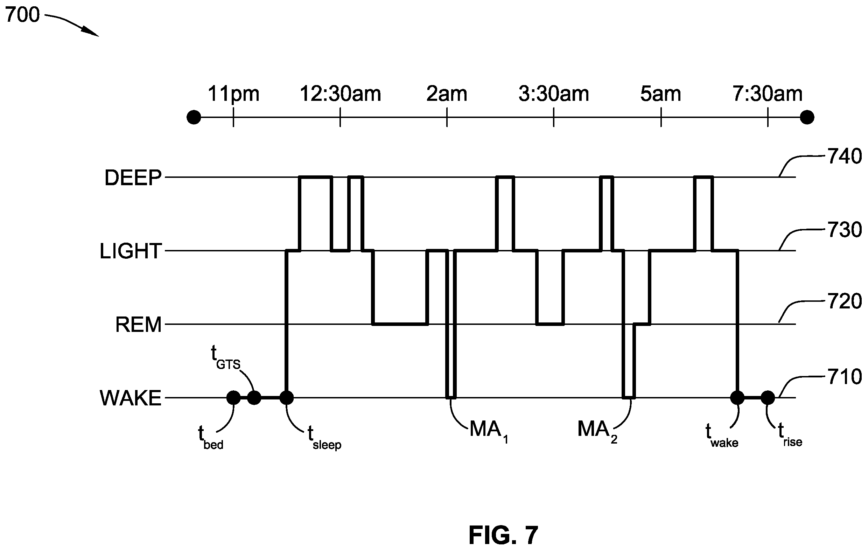

Referring to FIG. 7, an exemplary hypnogram 700 corresponding to the timeline 600 (FIG. 6), according to some implementations, is illustrated. As shown, the hypnogram 700 includes a sleep-wake signal (in bold), a wakefulness stage axis 710, a REM stage axis 720, a light sleep stage axis 730, and a deep sleep stage axis 740. The intersection between the sleep-wake signal and one of the axes 710-740 is indicative of the sleep stage at any given time during the sleep session.

The sleep-wake signal can be generated based on physiological data associated with the user (e.g., generated by one or more of the sensors 130 described herein). The sleep-wake signal can be indicative of one or more sleep states, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 700 is shown in FIG. 7 as including the light sleep stage axis 730 and the deep sleep stage axis 740, in some implementations, the hypnogram 700 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnogram 700 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (t_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 7), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the external device 170 (e.g., data indicative of the user no longer using the external device 170), data from the pressure sensor 132 and/or the flow rate sensor 134 (e.g., data indicative of the user turning on the respiratory therapy device 122, data indicative of the user donning the user interface 124, etc.), or any combination thereof.

One or more elements or aspects or steps, or any portion(s) thereof, from one or more of any of claims 1 to 68 below can be combined with one or more elements or aspects or steps, or any portion(s) thereof, from one or more of any of the other claims 1 to 68 or combinations thereof, to form one or more additional implementations and/or claims of the present disclosure.

## Claims

1. A method comprising:
generating, by a sensor, data including current data that is associated with a current sleep session of a plurality of sleep sessions and historical data that is associated with one or more prior sleep sessions of the plurality of sleep sessions;
analyzing the historical data to determine a behavior pattern associated with a user;
analyzing the current data to determine a condition of a user interface in relation to the user, wherein a respiratory therapy device is coupled to the user interface and is configured to supply pressurized air to an airway of the user via the user interface during the plurality of sleep sessions; and
generating an alarm based on the behavior pattern associated with the user and the condition of the user interface in relation to the user, the behavior pattern describing prior sleeping patterns of the user and prior usage of the respiratory therapy device by the user.

2. The method of claim 1, wherein the condition of the user interface in relation to the user includes an indication of whether the user interface is securely engaged to the user, or an indication that the user interface has been disengaged from the user for a predetermined amount of time.

3. The method of claim 2, further comprising:
determining the predetermined amount of time based at least in part on the current data and/or
determining the predetermined amount of time based at least in part on the behavior pattern associated with the user.

4. The method of any one of claims 1 to 3, wherein the user interface is a facial mask, a full-face mask, a nasal mask, or a nasal pillow mask.

5. The method of any one of claims 1 to 4, wherein the generating the alarm includes pulsing a flow of the supplied pressurized air for a period of time such that the user interface intermittently emits a sound during the period of time, wherein optionally the period of time is 10 seconds or the period of time is any amount of time between 2 and 30 seconds.

6. The method of any one of claims 1 to 5, further comprising:
setting a parameter of the alarm, the parameter of the alarm including: (a) a nature of the alarm, the nature of the alarm including a type of sound, a type of vibration, a type of notification, a type of light, or any combination thereof, (b) a magnitude of the alarm, the magnitude including a volume of the type of sound, an intensity of the type of vibration, an intensity of the type of light, or any combination thereof, (c) a duration of the alarm, (d) a pattern in modulating the amplitude of the alarm during at least a portion of the duration of the alarm, (e) a frequency of the alarm, (f) a pattern in modulating the frequency of the alarm during at least a portion of the duration of the alarm, (g) a period for re-triggering the alarm, or (h) any combination thereof,
wherein, optionally, the method further comprises adjusting the alarm, according to the set parameters, wherein the adjusting includes starting the alarm, varying the alarm, stopping the alarm, or any combination thereof, and/or
adjusting at least one of the set parameters of the alarm depending on a response of the user to the alarm.

7. The method of any one of claims 1 to 6, further comprising:
generating a report for the current sleep session, the report including a number of generated alarms during the current sleep session, a nature of each of the generated alarms, and a user response to each of the generated alarms,
wherein, optionally, the user response includes the user dismissing one or more of the generated alarms, the user ignoring one or more of the generated alarms, the user re-engaging with the user interface, the type of engagement of the user with the user interface, or any combination thereof.

8. The method of any one of claims 1 to 7, wherein the generating the alarm includes causing a speaker to emit a sound,
wherein, optionally, the emitted sound includes relaxing sounds, and wherein the relaxing sounds serve as reminders for the user to re-engage with the user interface and also serve as sleep aids to help the user fall back asleep.

9. The method of any one of claims 1 to 8, further comprising:
determining that the user has interacted with a mobile device after disengaging with the user interface, and wherein the generating the alarm includes sending a notification to the mobile device,
wherein, optionally, the notification is a reminder to the user to re-engage with the user interface, and/or
the determining that the user has interacted with the mobile device includes detecting a use of a screen of the mobile device, detecting a change in position of the mobile device, or both, and/or
the notification is a push notification.

10. The method of any one of claims 1 to 9, wherein the behavior pattern associated with the user includes one or more events associated with the user, the one or more events including: (i) the user waking up one or more times during at least one of the one or more prior sleep sessions, (ii) the user not engaging with the user interface during at least one of the one or more prior sleep sessions, (iii) the user not engaging with the user interface for a second portion, occurring after a first portion and after the user wakes up, of at least one of the one or more prior sleep sessions, (iv) the user having the user interface disengaged for a length of time during at least one of the one or more prior sleep session, before engaging with the user interface again during the same sleep session, (v) the user experiencing an apnea event during the one or more prior sleep sessions, (vi) the user being in a specific sleep stage or a sequence of sleep stages during at least one of the one or more prior sleep sessions, prior to the user disengaging the user interface and not engaging with the user interface for a subsequent portion of the at least one of the one or more prior sleep sessions, (vii) the user being in a specific sleep stage or a combination of sleep stages during at least one of the one or more prior sleep sessions, prior to the user having the user interface disengaged for a length of time during the at least one of the one or more prior sleep sessions, before engaging with the user interface again during the same sleep session, (viii) the user having an average waking time, or (ix) any combination thereof,
wherein, optionally, the generating the alarm is based at least in part on a type of the at least one of the one or more events, a frequency of at least one of the one or more events, a time of day of occurrence of the at least one of the one or more events, or any combination thereof.

11. The method of any one of claims 1 to 10, wherein the generating the alarm is further based at least in part on a predicted remaining duration of the current sleep session, and/or
wherein the generating the alarm is further based at least in part on the condition of the user interface indicating that the user interface is not securely engaged to the user and the predicted remaining duration of the current sleep session satisfying a threshold, and/or
determining a likelihood that the user will experience an apnea event during the predicted remaining duration of the current sleep session, and wherein the generating the alarm is further based at least in part on the condition of the user interface indicating that the user interface is not securely engaged to the user and the determined likelihood satisfying a threshold.

12. The method of any one of claims 1 to 11, wherein the condition of the user interface is determined based at least in part on a pulsing of the supplied pressurized air, and/or
wherein the sensor includes one or more microphones, one or more speakers, one or more radio frequency (RF) transmitters, one or more RF receivers, one or more ultra-wide band (UWB) transmitters, one or more UWB receivers, one or more cameras, one or more flow sensors, one or more air pressure sensors, one or more pressure pads, one or more actigraphy sensors, one or more motion sensors, one or more temperature sensors, one or more pulse sensors, one or more PPG sensors, or any combination thereof.

13. The method of any one of claims 1 to 12, further comprising:
generating additional data associated with the user, the additional data being indicative of the user experiencing one or more events including central apneas, obstructive apneas, mixed apneas, hypopneas, snoring, periodic limb movement, restless leg syndrome, or any combination thereof, and wherein the generating the alarm is based at least in part on the generated additional data, and/or
wherein the condition of the user interface indicates that the user interface is not currently engaging the user and wherein the method further comprises:
analyzing the historical data to determine a historical sleep metric associated with the user, the historical sleep metric being indicative of how well the user has slept in the past, the historical sleep metric including an average number of times the one or more prior sleep sessions of the user are interrupted; and wherein the generating the alarm is further based at least in part on the historical sleep metric satisfying a threshold.

14. The method of any one of claims 1 to 13, further comprising:
determining that the condition of the user interface changed from the user interface not being securely engaged to the user to the user interface being securely engaged to the user; and responsive to the determination that the user interface is securely engaged to the user: stop the alarm; cause the respiratory therapy device to provide the supplied pressurized air at a first pressure, the first pressure being relatively lower than a prescribed treatment pressure for the user; and ramp up the pressure of the supplied pressurized air from the first pressure to the prescribed treatment pressure over a predetermined time period.

15. A system comprising:
a control system comprising one or more processors; and
a memory having stored thereon machine readable instructions;
wherein the control system is coupled to the memory, and the method of any one of claims 1 to 14 is implemented when the machine executable instructions in the memory are executed by at least one of the one or more processors of the control system.

## Patentansprüche

1. Verfahren, umfassend:
Erzeugen von Daten durch einen Sensor, die aktuelle Daten umfassen, die mit einer aktuellen Schlafsitzung einer Mehrzahl von Schlafsitzungen assoziiert sind, und historischen Daten, die mit einer oder mehreren vorherigen Schlafsitzungen der Mehrzahl von Schlafsitzungen assoziiert sind;
Analysieren der historischen Daten, um ein Verhaltensmuster zu bestimmen, das mit einem Benutzer assoziiert ist;
Analysieren der aktuellen Daten, um einen Zustand einer Benutzerschnittstelle in Bezug auf den Benutzer zu bestimmen, wobei eine Atemtherapievorrichtung mit der Benutzerschnittstelle gekoppelt und dazu ausgelegt ist, einem Atemweg des Benutzers während der Mehrzahl von Schlafsitzungen Druckluft über die Benutzerschnittstelle zuzuführen; und
Erzeugen eines Alarms basierend auf dem mit dem Benutzer assoziierten Verhaltensmuster und dem Zustand der Benutzerschnittstelle in Bezug auf den Benutzer, wobei das Verhaltensmuster vorherige Schlafmuster des Benutzers und vorherige Nutzung der Atemtherapievorrichtung durch den Benutzer beschreibt.

2. Verfahren nach Anspruch 1, wobei der Zustand der Benutzerschnittstelle in Bezug auf den Benutzer eine Anzeige dessen, ob die Benutzerschnittstelle dem Benutzer sicher angelegt ist, oder eine Anzeige umfasst, dass die Benutzerschnittstelle für eine vorbestimmte Zeitdauer von dem Benutzer abgenommen wurde.

3. Verfahren nach Anspruch 2, ferner umfassend:
Bestimmen der vorbestimmten Zeitdauer zumindest teilweise basierend auf den aktuellen Daten und/oder
Bestimmen der vorbestimmten Zeitdauer zumindest teilweise basierend auf dem mit dem Benutzer assoziierten Verhaltensmuster.

4. Verfahren nach einem der Ansprüche 1 bis 3, wobei die Benutzerschnittstelle eine Gesichtsmaske, eine Vollgesichtsmaske, eine Nasenmaske oder eine Nasenpolstermaske ist.

5. Verfahren nach einem der Ansprüche 1 bis 4, wobei das Erzeugen des Alarms Pulsieren eines Stroms der zugeführten Druckluft für einen Zeitraum umfasst, sodass die Benutzerschnittstelle während des Zeitraums periodisch einen Ton ausgibt, wobei optional der Zeitraum 10 Sekunden beträgt oder der Zeitraum eine Zeitdauer zwischen 2 und 30 Sekunden ist.

6. Verfahren nach einem der Ansprüche 1 bis 5, ferner umfassend:
Festlegen eines Parameters des Alarms, wobei der Parameterdes Alarms Folgendes umfasst: (a) eine Beschaffenheit des Alarms, wobei die Beschaffenheit des Alarms eine Art von Ton, eine Art von Vibration, eine Art von Benachrichtigung, eine Art von Licht oder eine beliebige Kombination davon umfasst, (b) eine Stärke des Alarms, wobei die Stärke eine Lautstärke der Art von Ton, eine Intensität der Art von Vibration, eine Intensität der Art von Licht oder eine beliebige Kombination davon umfasst, (c) eine Dauer des Alarms, (d) ein Muster beim Modulieren der Amplitude des Alarms während zumindest eines Teils der Dauer des Alarms, (e) eine Häufigkeit des Alarms, (f) ein Muster beim Modulieren der Häufigkeit des Alarms während zumindest eines Teils der Dauer des Alarms, (g) einen Zeitraum zum erneuten Auslösen des Alarms oder (h) eine beliebige Kombination davon,
wobei das Verfahren optional ferner Anpassen des Alarms gemäß den festgelegten Parametern umfasst, wobei das Anpassen Starten des Alarms, Variieren des Alarms, Stoppen des Alarms oder eine beliebige Kombination davon umfasst,
Anpassen mindestens eines der festgelegten Parameterdes Alarms in Abhängigkeit von einer Reaktion des Benutzers auf den Alarm.

7. Verfahren nach einem der Ansprüche 1 bis 6, ferner umfassend:
Erstellen eines Berichts für die aktuelle Schlafsitzung, wobei der Bericht eine Anzahl erzeugter Alarme während der aktuellen Schlafsitzung, eine Beschaffenheit jedes der erzeugten Alarme und eine Benutzerreaktion auf jeden der erzeugten Alarme umfasst,
wobei die Benutzerreaktion optional Verwerfen eines oder mehrerer der erzeugten Alarme durch den Benutzer, Ignorieren eines oder mehrerer der erzeugten Alarme durch den Benutzer, erneutes Anlegen der Benutzerschnittstelle durch den Benutzer, die Art des Anlegens der Benutzerschnittstelle durch den Benutzer oder eine beliebige Kombination davon umfasst.

8. Verfahren nach einem der Ansprüche 1 bis 7, wobei das Erzeugen des Alarms Veranlassen eines Lautsprechers zum Ausgeben eines Tons umfasst,
wobei optional der ausgegebene Ton entspannende Töne umfasst, und wobei die entspannenden Töne dem Benutzer als Erinnerung dienen, die Benutzerschnittstelle erneut anzulegen, und außerdem als Einschlafhilfe dienen, um dem Benutzer dabei zu helfen, wieder einzuschlafen.

9. Verfahren nach einem der Ansprüche 1 bis 8, ferner umfassend:
Bestimmen, dass der Benutzer nach dem Abnehmen der Benutzerschnittstelle mit einer mobilen Vorrichtung interagiert hat, und wobei das Erzeugen des Alarms Senden einer Benachrichtigung an die mobile Vorrichtung umfasst,
wobei optional die Benachrichtigung eine Erinnerung für den Benutzer ist, die Benutzerschnittstelle erneut anzulegen, und/oder
das Bestimmen, dass der Benutzer mit der mobilen Vorrichtung interagiert hat, Erkennen einer Verwendung eines Bildschirms der mobilen Vorrichtung, Erkennen einer Änderung der Position der mobilen Vorrichtung oder beides umfasst, und/oder
die Benachrichtigung eine Push-Benachrichtigung ist.

10. Verfahren nach einem der Ansprüche 1 bis 9, wobei das mit dem Benutzer assoziierte Verhaltensmuster ein oder mehrere mit dem Benutzer assoziierte Ereignisse umfasst, wobei das eine oder die mehreren Ereignisse Folgendes umfassen: (i) der Benutzer wacht während mindestens einer der einen oder der mehreren vorherigen Schlafsitzungen ein- oder mehrmals auf, (ii) der Benutzer legt die Benutzerschnittstelle während mindestens einer der einen oder der mehreren vorherigen Schlafsitzungen nicht an, (iii) der Benutzer legt die Benutzerschnittstelle für einen nach einem ersten Teil und nach dem Aufwachen des Benutzers auftretenden zweiten Teil mindestens einer der einen oder der mehreren vorherigen Schlafsitzungen nicht an, (iv) der Benutzer hat die Benutzerschnittstelle für eine Zeitdauer während mindestens einer der einen oder der mehreren vorherigen Schlafsitzungen abgenommen, bevor er die Benutzerschnittstelle während derselben Schlafsitzung erneut anlegt, (v) der Benutzer erleidet während der einen oder mehreren vorherigen Schlafsitzungen ein Apnoe-Ereignis, (vi) der Benutzer befindet sich während mindestens einer der einen oder der mehreren vorherigen Schlafsitzungen in einer spezifischen Schlafphase oder einer Folge von Schlafphasen, bevor der Benutzer die Benutzerschnittstelle abnimmt und die Benutzerschnittstelle für einen nachfolgenden Teil der mindestens einen der einen oder der mehreren vorherigen Schlafsitzungen nicht mehr anlegt, (vii) der Benutzer befindet sich während mindestens einer der einen oder der mehreren vorherigen Schlafsitzungen in einer spezifischen Schlafphase oder einer Kombination von Schlafphasen, bevor der Benutzer die Benutzerschnittstelle für eine Zeitdauer während der mindestens einen der einen oder der mehreren vorherigen Schlafsitzungen abgenommen hat, bevor er während derselben Schlafsitzung die Benutzerschnittstelle erneut anlegt, (viii) die durchschnittliche Wachzeit des Benutzers oder (ix) eine beliebige Kombination davon,
wobei optional das Erzeugen des Alarms zumindest teilweise auf einer Art des mindestens einen des einen oder der mehreren Ereignisse, einer Häufigkeit des mindestens einen des einen oder der mehreren Ereignisse, einer Tageszeit des Eintretens des mindestens einen des einen oder der mehreren Ereignisse oder einer beliebigen Kombination davon basiert.

11. Verfahren nach einem der Ansprüche 1 bis 10, wobei das Erzeugen des Alarms ferner zumindest teilweise auf einer vorhergesagten verbleibenden Dauer der aktuellen Schlafsitzung basiert, und/oder
wobei das Erzeugen des Alarms ferner zumindest teilweise auf dem Zustand der Benutzerschnittstelle basiert, der anzeigt, dass die Benutzerschnittstelle dem Benutzer nicht sicher angelegt ist, und die vorhergesagte verbleibende Dauer der aktuellen Schlafsitzung einen Schwellenwert erreicht, und/oder
Bestimmen einer Wahrscheinlichkeit, dass der Benutzer ein Apnoe-Ereignis während der vorhergesagten verbleibenden Dauer der aktuellen Schlafsitzung erleidet, und wobei das Erzeugen des Alarms ferner zumindest teilweise auf dem Zustand der Benutzerschnittstelle basiert, der anzeigt, dass die Benutzerschnittstelle dem Benutzer nicht sicher angelegt ist und die bestimmte Wahrscheinlichkeit einen Schwellenwert erreicht.

12. Verfahren nach einem der Ansprüche 1 bis 11, wobei der Zustand der Benutzerschnittstelle zumindest teilweise basierend auf einem Pulsieren der zugeführten Druckluft bestimmt wird, und/oder
wobei der Sensor ein oder mehrere Mikrofone, einen oder mehrere Lautsprecher, einen oder mehrere Hochfrequenzsender (HF-Sender), einen oder mehrere HF-Empfänger, einen oder mehrere Ultrabreitbandsender (UWB-Sender), einen oder mehrere UWB-Empfänger, eine oder mehrere Kameras, einen oder mehrere Durchflusssensoren, einen oder mehrere Luftdrucksensoren, ein oder mehrere Druckpolster, einen oder mehrere Aktigraphiesensoren, einen oder mehrere Bewegungssensoren, einen oder mehrere Temperatursensoren, einen oder mehrere Impulsgeber, einen oder mehrere PPG-Sensoren oder eine beliebige Kombination davon umfasst.

13. Verfahren nach einem der Ansprüche 1 bis 12, ferner umfassend:
Erzeugen zusätzlicher Daten, die mit dem Benutzer assoziiert sind, wobei die zusätzlichen Daten anzeigen, dass der Benutzer ein oder mehrere Ereignisse erleidet, darunter zentrale Apnoen, obstruktive Apnoen, gemischte Apnoen, Hypopnoen, Schnarchen, periodische Gliedmaßenbewegungen, Restless-Legs-Syndrom oder eine beliebige Kombination davon, und wobei das Erzeugen des Alarms zumindest teilweise auf den erzeugten zusätzlichen Daten basiert, und/oder
wobei der Zustand der Benutzerschnittstelle anzeigt, dass die Benutzerschnittstelle dem Benutzer gegenwärtig nicht angelegt ist, und wobei das Verfahren ferner Folgendes umfasst:
Analysieren der historischen Daten, um eine mit dem Benutzer assoziierte historische Schlafmetrik zu bestimmen, wobei die historische Schlafmetrik anzeigt, wie gut der Benutzer in der Vergangenheit geschlafen hat, die historische Schlafmetrik eine durchschnittliche Anzahl von Unterbrechungen der einen oder der mehreren vorherigen Schlafsitzungen des Benutzers umfasst; und wobei das Erzeugen des Alarms ferner zumindest teilweise darauf basiert, dass die historische Schlafmetrik einen Schwellenwert erreicht.

14. Verfahren nach einem der Ansprüche 1 bis 13, ferner umfassend:
Bestimmen, dass sich der Zustand der Benutzerschnittstelle von "die Benutzerschnittstelle ist dem Benutzer nicht sicher angelegt" zu "die Benutzerschnittstelle ist dem Benutzer sicher angelegt" geändert hat; und in Reaktion auf die Bestimmung, dass die Benutzerschnittstelle dem Benutzer sicher angelegt ist:
Beenden des Alarms; Veranlassen der Atemtherapievorrichtung, die zugeführte Druckluft mit einem ersten Druck bereitzustellen, wobei der erste Druck relativ niedriger ist als ein verschriebener Behandlungsdruck für den Benutzer; und Erhöhen des Drucks der zugeführten Druckluft vom ersten Druckauf den verschriebenen Behandlungsdruck über einen vorbestimmten Zeitraum.

15. System, umfassend:
ein Steuersystem, das einen oder mehrere Prozessoren umfasst; und
einen Speicher, der maschinenlesbare Anweisungen darauf gespeichert aufweist;
wobei das Steuersystem mit dem Speicher gekoppelt ist und das Verfahren nach einem der Ansprüche 1 bis 14 implementiert wird, wenn die maschinenausführbaren Anweisungen in dem Speicher von mindestens einem des einen oder der mehreren Prozessoren des Steuersystems ausgeführt werden.

## Revendications

1. Procédé comprenant :
la génération, par un capteur, de données incluant des données actuelles qui sont associées à une session de sommeil actuelle d'une pluralité de sessions de sommeil et des données historiques qui sont associées à une ou plusieurs sessions de sommeil antérieures de la pluralité de sessions de sommeil ;
l'analyse des données historiques pour déterminer un modèle de comportement associé à un utilisateur ;
l'analyse des données actuelles pour déterminer l'état d'une interface utilisateur par rapport à l'utilisateur, où un dispositif de thérapie respiratoire est couplé à l'interface utilisateur et est configuré pour fournir de l'air sous pression aux voies respiratoires de l'utilisateur via l'interface utilisateur pendant la pluralité de sessions de sommeil ; et
la génération d'une alarme sur la base du modèle de comportement associé à l'utilisateur et de l'état de l'interface utilisateur par rapport à l'utilisateur, le modèle de comportement décrivant les modèles de sommeil antérieurs de l'utilisateur et l'utilisation antérieure du dispositif de thérapie respiratoire par l'utilisateur.

2. Procédé selon la revendication 1, dans lequel l'état de l'interface utilisateur par rapport à l'utilisateur inclut une indication pour savoir si l'interface utilisateur est solidement engagée avec l'utilisateur, ou une indication selon laquelle l'interface utilisateur a été désengagée de l'utilisateur pour un laps de temps prédéterminé.

3. Procédé selon la revendication 2, comprenant en outre :
la détermination du laps de temps prédéterminé sur la base au moins en partie des données actuelles et/ou
la détermination du laps de temps prédéterminé sur la base au moins en partie du modèle de comportement associé à l'utilisateur.

4. Procédé selon l'une quelconque des revendications 1 à 3, dans lequel l'interface utilisateur est un masque facial, un masque facial complet, un masque nasal ou un masque narinaire.

5. Procédé selon l'une quelconque des revendications 1 à 4, dans lequel la génération de l'alarme inclut la pulsation d'un flux de l'air sous pression fourni pendant une période de temps de sorte que l'interface utilisateur émette par intermittence un son pendant la période de temps, où éventuellement la période de temps est de 10 secondes ou la période de temps est un laps de temps quelconque entre 2 et 30 secondes.

6. Procédé selon l'une quelconque des revendications 1 à 5, comprenant en outre :
la définition d'un paramètre de l'alarme, le paramètre de l'alarme incluant : (a) une nature de l'alarme, la nature de l'alarme incluant un type de son, un type de vibration, un type de notification, un type de lumière, ou toute combinaison de ceux-ci, (b) une ampleur de l'alarme, l'ampleur incluant un volume du type de son, une intensité du type de vibration, une intensité du type de lumière, ou toute combinaison de ceux-ci, (c) une durée de l'alarme, (d) un modèle dans la modulation de l'amplitude de l'alarme pendant au moins une partie de la durée de l'alarme, (e) une fréquence de l'alarme, (f) un modèle dans la modulation de la fréquence de l'alarme pendant au moins une partie de la durée de l'alarme, (g) une période pour le redéclenchement de l'alarme, ou (h) toute combinaison de ceux-ci,
dans lequel, éventuellement, le procédé comprend en outre le réglage de l'alarme, selon les paramètres définis, où le réglage inclut le démarrage de l'alarme, la variation de l'alarme, l'arrêt de l'alarme, ou toute combinaison de ceux-ci, et/ou
le réglage d'au moins l'un des paramètres définis de l'alarme en fonction d'une réponse de l'utilisateur à l'alarme.

7. Procédé selon l'une quelconque des revendications 1 à 6, comprenant en outre :
la génération d'un rapport pour la session de sommeil actuelle, le rapport incluant un certain nombre d'alarmes générées pendant la session de sommeil actuelle, une nature de chacune des alarmes générées et une réponse d'utilisateur à chacune des alarmes générées,
dans lequel, éventuellement, la réponse d'utilisateur inclut le rejet par l'utilisateur d'une ou de plusieurs des alarmes générées, l'ignorance par l'utilisateur d'une ou de plusieurs des alarmes générées, le réengagement de l'utilisateur avec l'interface utilisateur, le type d'engagement de l'utilisateur avec l'interface utilisateur, ou toute combinaison de ceux-ci.

8. Procédé selon l'une quelconque des revendications 1 à 7, dans lequel la génération de l'alarme inclut le fait d'amener un haut-parleur à émettre un son,
dans lequel, éventuellement, le son émis inclut des sons relaxants, et dans lequel les sons relaxants servent de rappels pour l'utilisateur pour se réengager avec l'interface utilisateur et servent également d'aides au sommeil pour aider l'utilisateur à se rendormir.

9. Procédé selon l'une quelconque des revendications 1 à 8, comprenant en outre :
la détermination du fait que l'utilisateur a interagi avec un dispositif mobile après le désengagement de l'interface utilisateur, et où la génération de l'alarme inclut l'envoi d'une notification au dispositif mobile,
dans lequel, éventuellement, la notification est un rappel à l'utilisateur pour se réengager avec l'interface utilisateur, et/ou
la détermination du fait que l'utilisateur a interagi avec le dispositif mobile inclut la détection d'une utilisation d'un écran du dispositif mobile, la détection d'un changement de position du dispositif mobile, ou les deux, et/ou
la notification est une notification poussée.

10. Procédé selon l'une quelconque des revendications 1 à 9, dans lequel le modèle de comportement associé à l'utilisateur inclut un ou plusieurs événements associés à l'utilisateur, le ou les plusieurs événements incluant : (i) le réveil de l'utilisateur une ou plusieurs fois pendant au moins l'une de la ou des plusieurs sessions de sommeil antérieures, (ii) l'absence d'engagement de l'utilisateur avec l'interface utilisateur pendant au moins l'une de la ou des plusieurs sessions de sommeil antérieures, (iii) l'absence d'engagement de l'utilisateur avec l'interface utilisateur pour une seconde partie, survenant après une première partie et après le réveil de l'utilisateur, d'au moins l'une de la ou des plusieurs sessions de sommeil antérieures, (iv) le fait que l'utilisateur présente l'interface utilisateur désengagée pour un délai pendant au moins l'une de la ou des plusieurs sessions de sommeil antérieures, avant l'engagement à nouveau avec l'interface utilisateur pendant la même session de sommeil, (v) le fait que l'utilisateur subit un événement d'apnée pendant la ou les plusieurs sessions de sommeil antérieures, (vi) le fait que l'utilisateur est dans un stade de sommeil spécifique ou une séquence de stades de sommeil pendant au moins l'une de la ou des plusieurs sessions de sommeil antérieures, avant que l'utilisateur ne se désengage de l'interface utilisateur et ne s'engage pas avec l'interface utilisateur pour une partie ultérieure de l'au moins une de la ou des plusieurs sessions de sommeil antérieures, (vii) le fait que l'utilisateur est dans un stade de sommeil spécifique ou une combinaison de stades de sommeil pendant au moins l'une de la ou des plusieurs sessions de sommeil antérieures, avant que l'utilisateur ne présente l'interface utilisateur désengagée pour un délai pendant l'au moins une de la ou des plusieurs sessions de sommeil antérieures, avant l'engagement à nouveau avec l'interface utilisateur pendant la même session de sommeil, (viii) le fait que l'utilisateur présente un temps de réveil moyen, ou (ix) toute combinaison de ceux-ci,
dans lequel, éventuellement, la génération de l'alarme est basée au moins en partie sur un type de l'au moins un du ou des plusieurs événements, une fréquence d'au moins l'un du ou des plusieurs événements, un moment de la journée d'occurrence de l'au moins un du ou des plusieurs événements, ou toute combinaison de ceux-ci.

11. Procédé selon l'une quelconque des revendications 1 à 10, dans lequel la génération de l'alarme est en outre basée au moins en partie sur la durée restante prévue de la session de sommeil actuelle, et/ou
dans lequel la génération de l'alarme est en outre basée au moins en partie sur l'état de l'interface utilisateur indiquant que l'interface utilisateur n'est pas solidement engagée avec l'utilisateur et la durée restante prévue de la session de sommeil actuelle satisfaisant un seuil, et/ou
la détermination de la probabilité que l'utilisateur subisse un événement d'apnée pendant la durée restante prévue de la session de sommeil actuelle, et où la génération de l'alarme est en outre basée au moins en partie sur l'état de l'interface utilisateur indiquant que l'interface utilisateur n'est pas solidement engagée avec l'utilisateur et la probabilité déterminée satisfaisant un seuil.

12. Procédé selon l'une quelconque des revendications 1 à 11, dans lequel l'état de l'interface utilisateur est déterminé sur la base au moins en partie d'une pulsation de l'air sous pression fourni, et/ou
dans lequel le capteur inclut un ou plusieurs microphones, un ou plusieurs haut-parleurs, un ou plusieurs émetteurs de radiofréquence (RF), un ou plusieurs récepteurs RF, un ou plusieurs émetteurs à bande ultra-large (UWB), un ou plusieurs récepteurs UWB, une ou plusieurs caméras, un ou plusieurs capteurs de débit, un ou plusieurs capteurs de pression d'air, un ou plusieurs coussinets de pression, un ou plusieurs capteurs d'actigraphie, un ou plusieurs capteurs de mouvement, un ou plusieurs capteurs de température, un ou plusieurs capteurs d'impulsions, un ou plusieurs capteurs PPG, ou toute combinaison de ceux-ci.

13. Procédé selon l'une quelconque des revendications 1 à 12, comprenant en outre :
la génération de données supplémentaires associées à l'utilisateur, les données supplémentaires indiquant que l'utilisateur subit un ou plusieurs événements incluant des apnées centrales, des apnées obstructives, des apnées mixtes, des hypopnées, des ronflements, un mouvement périodique des membres, le syndrome des jambes sans repos ou toute combinaison de ceux-ci, et où la génération de l'alarme est basée au moins en partie sur les données supplémentaires générées, et/ou
dans lequel l'état de l'interface utilisateur indique que l'interface utilisateur ne s'engage pas actuellement avec l'utilisateur et dans lequel le procédé comprend en outre :
l'analyse des données historiques pour déterminer une mesure de sommeil historique associée à l'utilisateur, la mesure de sommeil historique indiquant à quel point l'utilisateur a bien dormi dans le passé, la mesure de sommeil historique incluant un nombre moyen de fois où la ou les plusieurs sessions de sommeil antérieures de l'utilisateur sont interrompues ; et où la génération de l'alarme est en outre basée au moins en partie sur la mesure de sommeil historique satisfaisant un seuil.

14. Procédé selon l'une quelconque des revendications 1 à 13, comprenant en outre le fait :
de déterminer que l'état de l'interface utilisateur est passé de l'interface utilisateur non solidement engagée avec l'utilisateur à l'interface utilisateur solidement engagée avec l'utilisateur ; et en réponse à la détermination du fait que l'interface utilisateur est solidement engagée avec l'utilisateur : d'arrêter l'alarme ; d'amener le dispositif de thérapie respiratoire à fournir l'air sous pression fourni à une première pression, la première pression étant relativement inférieure à une pression de traitement prescrite pour l'utilisateur ; et d'augmenter la pression de l'air sous pression fourni de la première pression à la pression de traitement prescrite sur une période de temps prédéterminée.

15. Système comprenant :
un système de commande comprenant un ou plusieurs processeurs ; et
une mémoire sur laquelle sont stockées des instructions lisibles par machine ;
dans lequel le système de commande est couplé à la mémoire, et le procédé selon l'une quelconque des revendications 1 à 14 est mis en œuvre lorsque les instructions exécutables par machine dans la mémoire sont exécutées par au moins l'un du ou des plusieurs processeurs du système de commande.
